(19) Europäisches Patentamt
European Patent Office
Office européen des brevets

(11) Publication number: 0 006 739
B1

(12) **EUROPEAN PATENT SPECIFICATION**

(45) Date of publication of patent specification: 13.10.82

(51) Int. Cl.³: A 61 K 7/38

(21) Application number: 79301203.0

(22) Date of filing: 21.06.79

# (54) Antiperspirant composition and methods for its preparation and use.

(30) Priority: 23.06.78 GB 2775578

(43) Date of publication of application:
09.01.80 Bulletin 80/1

(45) Publication of the grant of the patent:
13.10.82 Bulletin 82/41

(84) Designated Contracting States:
AT CH DE FR GB IT NL SE

(56) References cited:
DE - A - 2 700 711
FR - A - 2 039 249
FR - A - 2 259 587
FR - A - 2 278 319

(73) Proprietor: UNILEVER PLC
Unilever House Blackfriars P O Box 68
London EC4P 4BQ (GB)
(84) GB
(73) Proprietor: UNILEVER NV
Burgemeester s'Jacobplein 1
NL-3000 DK Rotterdam (NL)
(84) CH DE FR IT NL SE AT

(72) Inventor: Gosling, Keith
Flat 5, 50152 Mount Ararat Road
Richmond, Surrey (GB)
Inventor: Mulley, Victor John
12 Selsdon Avenue
Woodley Reading, Berkshire (GB)
Inventor: Baldock, Michael John
17 Shrewsbury Walk
Isleworth, Middlesex (GB)

(74) Representative: Doucy, Robert Henry et al,
Unilever PLC, Patent Division PO Box 31 Salisbury
Square House Salisbury Square
London EC4 4AN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 006 739 B1

# Antiperspirant composition and methods for its preparation and use

This invention relates to the inhibition of perspiration by the application to the skin of antiperspirant active compounds.

In our copending German application P27 00 711.0 we have described basic aluminium chloride, bromide, iodide and nitrate compounds having enhanced antiperspirant activity. Although basic aluminium compounds are well known to have antiperspirant activity it has been shown in the copending application referred to that an enhancement in the antiperspirant activity of basic aluminium compounds having an aluminium to chloride, bromide, iodide or nitrate molar ratio of from 1.3 to 6.5:1 can be obtained by prolonged heating of aqueous solutions of said compounds under certain conditions leading to the formation of higher polymeric species having a size above 100 Ångstroms. The antiperspirant active compounds having enhanced activity are defined in the said copending application as those which in water form an aqueous solution in which there is at least 2% by weight of the total aluminium contained in polymeric species having a size greater than 100 Ångstroms. The copending application shows that various factors are important in determining whether and at what rate the defined higher polymeric species are formed. One factor of importance is the concentration of the aqueous solution of the basic aluminium compound; increasing concentration acting to reduce the rate of production of polymers having a size greater than 100 Ångstroms. Data given in the copending application show that antiperspirant activity of the basic aluminium compound increased as the content of the higher polymers in the heat treated solution increased up to a point at which between about 40% and 60% of the aluminium was contained in polymers having a size above 100 Ångstroms. Commercially available basic aluminium compounds, and in particular those sold for antiperspirant use, do not, so far as is known, contain in aqueous solution polymers having an effective diameter above 100 Ångstroms.

We have now made a further discovery concerning the characterisation of basic aluminium chloride, bromide and iodide compounds which have especially good antiperspirant activity which does not depend on the requirement that their aqueous solution should contain at least 2% of the aluminium contained in polymers exceeding 100 Ångstroms in effective diameter, and indeed they may in aqueous solution contain substantially no polymers of such size.

The basic aluminium compounds employed in the present invention for inhibiting perspiration, have an aluminium to chloride, bromide or iodide molar ratio of from 0.5 to 2.5:1 and have such size distribution of polymeric species that in the size exclusion chromatographic procedure described herein there is eluted a fraction between the relative retention times of 0.76 and 0.82 in which fraction there is contained at least 20% of the total aluminium of the compound. For convenience, such fraction is referred to hereinafter as the Band III fraction.

According to the present invention, therefore, there is provided a method of inhibiting perspiration comprising applying to the skin a polymeric antiperspirant compound which is a basic aluminium chloride, bromide or iodide having an aluminium to chloride, bromide or iodide molar ratio of from 0.5 to 2.5:1 in the form of a powder or an aqueous solution having an aluminium concentration of at least 2.5% by weight, the basic compound having at least 20% of the total aluminium in the Band III fraction (as defined hereinafter) and less than 2% of the total aluminium contained in polymers having a size exceeding 100 Ångstroms. The amount of aluminium contained in the Band III fraction is preferably at least 25%, more preferably at least 30%, and may even exceed 80% of the total aluminium.

Characterisation of materials containing species differing in size by means of size exclusion chromatography is generally known. The size exclusion chromatographic procedure for characterising the basic aluminium compounds of this invention will now be described.

The analytical procedure is performed on a stainless steel column of dimensions 30 cm high and of 7 mm internal diameter packed with porous silica of nominal particle size 5 microns and pore size of 60 Angstroms, which silica has been deactivated by silylation to eliminate adsorption in size exclusion separations. A suitable silica is that available commercially as LiChrosorb RP—2. The silica employed by the Applicants in deriving analytical data given herein had a cumulative undersize particle size distribution by weight of 10% less than 5 microns, 50% less than 6 microns and 90% less than 7 microns.

The column is fitted at the bottom with a zero dead volume fitting containing a 2 micron mesh stainless steel bed support. The silica is packed into the column by the high pressure slurry method (see Practical High Performance Liquid Chromatography, Edited by C.F. Simpson, 1976, Appendix II), using methanol:water (90:10) containing 1% sodium acetate as the packing medium.

After packing, the column is capped with another zero dead volume fitting containing a 2 micron stainless steel mesh. The packed column is then eluted with 200 ml of methanol at a flow rate of about 10 ml/min, using a high pressure pump, to consolidate the bed and wash out the packing medium. The bed is topped up, if necessary, with a thick slurry of the packing in methanol followed by reconsolidation.

A differential refractive index monitor (e.g. Waters R401), is used to detect sample fractions as they are eluted. It is linked to a pen recorder to provide a chromatogram and to an integrator (e.g.

Infotronics CRS 309) which measures the elution times of the fractions and the relative chromatographic band areas. The integrator is required to measure areas of bands not resolved to the baseline by dropping perpendiculars from the lowest point of the valleys separating the bands to the baseline.

The column packing should be tested according to the procedure of Bristow & Knox (Chromatographia, Volume 10, No. 6, June 1977, pp 279—89) for reverse phase materials and should generate at least 20,000 plates/metre for the test component phenetole.

To prepare test solutions of the materials for analysis those already in solution are diluted, if necessary, with deionized water to give 2 g of a solution containing 2.5% by weight aluminium and dispersed by treatment with a sonic probe for 2 minutes. Solid materials (e.g. spray dried powders) are dissolved in deionized water to give 2 g of a solution containing 2.5% by weight aluminium and dispersed by treatment with a sonic probe for 2 minutes. The solutions prepared in this way are filtered through a 25 mm diameter membrane having a pore size of 0.025 micrometres to give the test solutions. The preparation of a test solution is carried out immediately prior to application of a sample thereof to the column.

A sample of the test solution containing about 4 micromoles of aluminium is applied to the top of the column by means of a precision micro-litre syringe and a sample injection port. The sample is eluted with a $1 \times 10^{-2}$ M aqueous nitric acid solution at a flow rate of 1.0 ml/min using a high pressure pump. The eluent is maintained at a temperature of 22—23°C.

Eluted fractions of a test sample are characterized by means of the ratio of their retention times to the retention time of the totally included species. In the case of basic aluminium chlorides the totally included species arises from hydrochloric acid (which is present in solutions of basic aluminium chlorides) as can be shown by comparison of its retention time with that of a sample of hydrochloric acid.

Using columns satisfying the above description and employing a standard solution of a basic aluminium chloride prepared as described below, the Applicants have obtained separation into four aluminium-containing fractions having relative retention times within the ranges indicated.

| | Band I | Band II | Band III | Band IV |
|---|---|---|---|---|
| Relative Retention Time Range | 0.62—0.70 | 0.71—0.75 | 0.76—0.82 | 0.83—0.97 |

The standard basic aluminium chloride solution is prepared as a solution containing 12.5% by weight aluminium from 19.1 g of aluminium chloride hexahydrate, 10.5 g of 99.9% pure aluminium wire (0.76 mm diameter, cut in approximately 1 cm lengths and degreased by washing in acetone) and 70.4 g of deionised water. The mixture is stirred and heated at 80—90°C under a reflux condenser until all of the aluminium is dissolved. Any traces of insoluble solids are removed by filtration to give a clear solution.

When this material is analysed by the size exclusion chromatographic procedure described herein, there are obtained the following four fractions having typical relative retention times and chromatographic band areas expressed as percentages of the total chromatographic band area representing aluminium-containing material.

| | Band I | Band II | Band III | Band IV |
|---|---|---|---|---|
| Relative Retention Time | 0.65 | 0.73 | 0.79 | 0.91 |
| Band Area % of total aluminium band area | 39 | 51 | 4 | 6 |

A standard basic aluminium bromide solution was prepared in a manner similar to that as described above for the chloride by employing 29.7 g aluminium bromide hexahydrate, 10.7 g aluminium wire and 59.6 g water. Analysis again gave four fractions having the relative retention times and chromatographic band areas expressed as percentages of the total chromatographic band area representing aluminium-containing material indicated below.

| | Band I | Band II | Band III | Band IV |
|---|---|---|---|---|
| Relative Retention Time | 0.62 | 0.74 | 0.80 | 0.94 |
| Band Area % of total aluminium band area | 34 | 56 | 7 | 3 |

The standard solutions contained 0% aluminium as polymers greater than 100 Angstroms in effective diameter.

It will be appreciated by those skilled in the art that mechanisms of separation other than the principal mechanism of size exclusion may play a part in this type of chromatography. Examples of the processes would be adsorption effects and hydrodynamic effects. Thus although it is possible for a given column and constant operating conditions to lead to invariable relative retention times, minor variations in particle size range and pore size distribution of the column packing material may lead to slight differences in relative retention times.

Quantitatively, the amount of aluminium in the Band III fraction expressed as a percentage of the total aluminium of the compound under test is readily determined from the area of its band on the chromatogram. This percentage is derived from the expression

$$\% \text{ Aluminium} = (100-A) \times \frac{\text{Area of band corresponding to Band III fraction}}{\text{Sum of the areas of the bands corresponding to the aluminium-containing fractions}}$$

where A is the percentage of the total aluminium which is contained in polymers greater than 100 Angstroms and is determined by the method described hereinafter.

In experiments performed by the Applicants using certain samples of test materials, the complete elution of all the applied aluminium in a sample was checked by direct analysis of another sample of the same volume by plasma emission spectrophotometry. The correlation between band area percentage and aluminium percentage was also verified by direct analysis. The fractions were collected as they emerged from the refractive index monitor and their individual aluminium contents measured also by plasma emission spectrophotometry.

The Applicants have analysed samples of a number of commercially available aluminium chlorhydrates recommended for use as antiperspirant agents. The percentage of aluminium in the Band III fraction for the tested samples is indicated below.

| | % Aluminium in Band III Fraction |
|---|---|
| 50% aqueous solution of aluminium chlorhydrate[1] | 6 |
| 50% aqueous solution of aluminium chlorhydrate[2] | 2 |
| Dried powder of aluminium chlorhydrate[3] | 8 |
| Dried powder of aluminium chlorhydrate[4] | 4 |
| Spray dried powder of aluminium chlorhydrate[5] | 10 |

[1] From Armour Pharmaceutical (Ireland) Limited as "Reheis Chlorhydrol".
[2] From Albright & Wilson.
[3] From Hoechst AG as Locron P.
[4] From Hoechst AG as Locron P extra.
[5] From Armour Pharmaceutical (Ireland) Limited as "Reheis aluminium chlorhydrate Microdry Ultrafine".

We have discovered that by modifying such materials so as to increase the aluminium content of the Band III fraction, the antiperspirant activity thereof is enhanced.

Such increase in activity may be brought about by heating aqueous solutions of the basic aluminium compound in the same way as described in the copending application referred to above and as will be set forth below. While enhancement of the percentage of aluminium in the Band III fraction in many cases is accompanied by the production of polymeric species having an effective diameter above 100 Ångstroms in which species a substantial proportion of the aluminium may be contained, we have found that this is not necessarily so. The present application is therefore more particularly concerned with the enhancement of antiperspirant efficacy by treatment conditions which do not result in 2% or more of the aluminium being present in polymers having a size greater than 100 Ångstroms.

One of the factors which has an effect on the production of polymers greater than 100 Angstroms is the acidity of the basic aluminium compound. As the acidity increases the rate of production of polymers above 100 Angstroms decreases. In the copending application the basic aluminium compounds in question were confined to those having an aluminium to chloride, bromide or iodide molar ratio of at least 1.3:1. With compounds having an aluminium to chloride, bromide or iodide molar ratio at or close to 1.3:1 there is the practical disadvantage that one requires to use relatively dilute solutions to produce polymers having a size above 100 Ångstroms and even then relatively high temperatures and/or long heating times may be necessary. We have found that even more acidic solutions can be modified to enhance their activity if they are treated so as to increase their Band III fraction. The invention of this application therefore is particularly useful when applied to basic aluminium compounds having an aluminium to chloride, bromide or iodide molar ratio of from 0.5 to 2.5:1, especially 1.0 to 2.2:1.

Apart from the choice of lower Al/Cl, Br or I molar ratios of 0.5 to 2.5, one can further favour the production of the polymeric species of the Band III fraction without production of polymers above 100 Angstroms in effective diameter through the use of more concentrated solutions. The aluminium concentration of the solution of the basic aluminium compound to be treated may range from 2.5% to 8.5%, preferably 3 to 6.5% by weight. The temperature at which the treatment of the solution is carried out is also significant. While low temperatures such as 50°C favour production of a product containing Band III polymers without any polymers above 100 Ångstroms, the rate of production of the polymeric species present in the Band III fraction is lower. Therefore, higher temperatures, up to about 140°C are preferable, production of polymers above 100 Angstroms being prevented or minimised by appropriate choice of the Al:Cl molar ratio (in the case of a basic aluminium chloride) and solution concentration. The time for which the treatment is carried out should be sufficient to produce a substantial enhancement of the Band III fraction. The time may range from 0.5 hour to 20 days.

The aqueous solution of the basic aluminium compound employed in the invention and characterised by a percentage of aluminium in the Band III fraction of at least 20% may be dried to give the compound in the form of a solid hydrate. As with solutions of conventionally used basic aluminium compounds, for example $^5/_6$ basic aluminium chloride, drying conditions which lead to both the loss of water of condensation, between the hydroxy groups of the compound, and acid should be avoided as these may lead to irreversible degradation of the treated basic aluminium compound. Any suitable method of drying may be used, spray drying being a particularly useful method. The spray drying method described in US Patent No. 3,887,692 may be employed. The solid material may be ground or milled as required.

The basic aluminium compounds employed in the present invention may be represented by the empirical formula

$$Al_2(OH)_{6-a} X_a$$

where X is Cl, Br or I and *a* is from 0.8 to 4.0, the formula in the case of the compound in solid form containing 0.5 to 8, preferably 0.5 to 4, molecules of water. Preferably the basic aluminium compound has an aluminium to chloride, bromide or iodide molar ratio of from 1 to 2.2:1.

In a further aspect the invention relates to a method of making an antiperspirant product which comprises incorporating in an applicator for applying an antiperspirant active compound to the skin a basic aluminium chloride, bromide or iodide having an aluminium to chloride, bromide or iodide molar ratio of from 0.5 to 2.5:1 in the form of a powder or an aqueous solution having an aluminium concentration of at least 2.5% by weight, the basic compound having at least 20% of the total aluminium in the Band III fraction and less than 2% of the total aluminium contained in polymers exceeding 100 Ångstroms.

The antiperspirant product may be one in which the applicator is a container fitted with a valve for dispensing liquid in aerosol form and the antiperspirant composition comprises a suspension of the antiperspirant active compound in particulate form in a liquid carrier which may be in admixture with a propellant. Furthermore, the product may be one wherein the applicator is a container fitted with a valve for dispensing liquid in aerosol form and the antiperspirant composition comprises an aqueous or aqueous alcoholic solution of said antiperspirant active compound. In this case the aqueous solution may be discharged by a propellant gas or by a finger-operated pump mechanism or by containing the composition within a container of pliable material whereby by squeezing the container the composition is expelled through the spray valve. Another form of the product is one in which the applicator is a roll-on applicator and the antiperspirant composition comprises an aqueous or aqueous alcoholic solution of the said antiperspirant active compound. Furthermore, the product may be one wherein the applicator is an applicator for dispensing a powdered material and the antiperspirant composition is a powdered composition including said antiperspirant active compound in powder form. The applicator may also be a stick applicator for holding an antiperspirant composition in the form of a stick or it may be tissue or cloth which is impregnated with the antiperspirant active material.

In accordance with another aspect of the invention there is provided a method of making an antiperspirant composition comprising forming a blend of a basic aluminium chloride, bromide or iodide

having an aluminium to chloride, bromide or iodide molar ratio of from 0.5 to 2.5:1, in the form of a powder or an aqueous solution having an aluminium concentration of at least 2.5% with a suitable adjunct or carrier material, the basic aluminium compound having at least 20% of the total aluminium in the Band III fraction and less than 2% of the total aluminium contained in polymers having a size exceeding 100 Ångstroms. The antiperspirant composition may be in the form of a lotion made by blending an aqueous or aqueous alcoholic solution of the basic aluminium compound having an aluminium concentration of from 2.5 to 7.5% by weight and 0.1 to 5% by weight of a thickening agent. Suitable thickening agents to antiperspirant lotions are well known to those skilled in the art, and include for example, magnesium aluminium silicates. Thickening may also be effected by emulsifying an oil or the like in the composition. Furthermore, the composition may comprise an aqueous or aqueous alcoholic solution of the basic aluminium compound having an aluminium concentration of from 2.5 to 7.5% by weight and from 0.1 to 1% by weight of perfume.

The composition may comprise an aqueous alcoholic solution of the basic aluminium compound containing from 1 to 60% by weight of an alcohol. These aqueous alcoholic compositions preferably contain ethanol or isopropanol as the alcohol which are preferably present in an amount of from about 1 to 30% by weight of the composition. Antiperspirant compositions comprising an aqueous solution of the active compound may contain from about 1 to 80% by weight of a propellant.

Aqueous or aqueous alcoholic solutions may contain a basic or neutral amino acid as described in German application No. 2818321 or urea to inhibit gelation.

The antiperspirant composition may also be made by blending a powdered antiperspirant active compound and a powdered inert solid diluent or organic liquid carrier, wherein said compound is a basic aluminium chloride, bromide or iodide having an aluminium to chloride, bromide or iodide molar ratio of from 0.5 to 2.5:1, which compound in the chromatographic test described herein exhibits a Band III fraction containing at least 20% of the total aluminium. The composition may be in the form of a powder aerosol composition comprising a suspension of the basic aluminium compound in particulate form in a liquid carrier, said composition also comprising a propellant. In particular the composition may be in the form of a powder aerosol composition comprising:

A. from about 1% to about 20% by weight of said basic aluminium compound in powder form;

B. from about 0.1% to about 5% by weight of a suspending agent;

C. from about 1% to about 25% by weight of a carrier liquid; and

D. from about 50% to about 96% by weight of a propellant.

The carrier liquid may for example be a non-volatile non-hygroscopic liquid as suggested in US Patent No. 3,968,203. Especially useful are carrier liquids which have emollient properties and a number of these are referred to in British Patent Specification No. 1,393,860. Especially preferred are fatty acid esters such as isopropyl myristate and those esters referred to in British Patent Specification No. 1,353,914 such as dibutyl phthalate and diisopropyl adipate.

Various other carrier liquids for powder suspension aerosols are suggested in US Patent Specifications Nos. 3,833,721, 3,833,720, 3,920,807, 3,949,066 and 3,974,270, and in British Patent Specifications Nos. 1,341,748, 1,300,260, 1,369,872 and 1,411,547. Volatile carrier liquids may also be used such as ethanol as described in South African Patent Specification No. 75/3576, and volatile silicones.

The ratio of total solids in the compositions to the carrier liquid may vary over a wide range, for example from 0.01 to 3 parts of the powder per part by weight of the carrier liquid.

The propellant can be a liquefied hydrocarbon, halogenated hydrocarbon or a mixture thereof. Examples of materials that are suitable for use as propellants are given in the above-mentioned patents and include trichlorofluoromethane, dichlorodifluoromethane, dichlorotetrafluoroethane, monochlorodifluoromethane, trichlorotrifluoroethane, propane, butane, 1,1-difluoroethane, 1,1-difluoro-1-chloroethane, dichloromonofluoromethane, methylene chloride, isopentane and isobutane, used singly or admixed. Trichlorofluoromethane, dichlorodifluoromethane, dichlorotetrafluoroethane, and isobutane, used singly or admixed, are preferred.

Examples of materials that are suitable for use as permanent gas propellants are nitrogen, carbon dioxide and nitrous oxide.

It is common practice to include in aerosol powder spray compositions a material to assist in the suspending of the powder in the liquid vehicle. The materials prevent compacting of the powder and they may also acts as thickening or gelling agents for the liquid vehicle. Especially preferred are hydrophobic clays and colloidal silicas. Hydrophobic clays are available under the trade name Bentone, e.g. Bentone 34 or Bentone 38, and their use as suspending agents are described in a number of patent specifications including US Patent Specification No. 3,773,683. Suitable colloidal silicas include those available under the trade marks Aerosil 200 and Cab-O-Sil M—5 as well as other grades.

The antiperspirant composition may, however, simply comprise from 5 to 40% by weight of said basic aluminium compound in powder form, the remainder consisting essentially of an inert powder material, such as talc or starch, for example.

The invention also relates to a novel antiperspirant compound comprising a hydrated basic aluminium chloride, bromide or iodide having an aluminium to chloride, bromide or iodide molar ratio of from 0.5 to 2.5:1 having at least 20% of the total aluminium in the Band III fraction and less than 2% of

the total aluminium contained in polymers having a size exceeding .100 Angstroms. The solid antiperspirant compound may have the empirical formula:

$$Al_2(OH)_{6-a}\ X_a nH_2O$$

where X is Cl, Br or I, *a* is from 0.8 to 4, preferably 0.9 to 2, and *n* is from 0.5 to 8. The solid compound preferably contains 0.5 to 4 molecules of water of hydration. A form of said solid compound particularly suitable for use in aerosol powder spray compositions is one comprising particles having a diameter less than 100 micrometer, preferably less than 74 micrometer.

In a further aspect, the invention relates to an aqueous solution of a basic aluminium chloride, bromide or iodide having an aluminium to chloride, bromide or iodide molar ratio of from 0.5 to 2.5:1 and having an aluminium content of from 2.5 to 8.5% by weight, the basic aluminium compound having at least 20% of the total aluminium in the Band III fraction and less than 2% of the total aluminium in polymers having a size exceeding 100 Ångstroms.

According to a further aspect of the invention there is provided a novel process for improving the antiperspirant activity of a basic aluminium chloride, bromide or iodide having an aluminium to chloride, bromide or iodide molar ratio of from 0.5 to 2.5:1, characterised in that an aqueous solution of the basic aluminium compound having an aluminium concentration of from 2.5 to 8.5% by weight is heated at 50°C to 140°C, said molar ratio, concentration and temperature and the time of heating being so chosen that the heat-treated product obtained has at least 20% of the total aluminium in the Band III fraction and less than 2% of the total aluminium in polymers having a size exceeding 100 Ångstroms. The solution may be dried, such as by spray drying, to produce the improved antiperspirant compound in solid form.

In the Examples given herein two test methods are referred to for the assessment of the antiperspirant efficacy of various antiperspirant active materials. Details of the test procedures are described below. The methods depend on subjecting human volunteers to thermal stress and gravimetric determination of axillar sweat.

Test Method 1

| | |
|---|---|
| Subjects | A panel of up to 54 women who use no antiperspirant for the 14 days before the test. |
| Hot Room | Temperatures 37°C ± 1°C, relative humidity approximately 35%. |
| Products | Two aerosol powder spray products of which one is designated the control. The panel is divided into two equal groups. One group receives the test treatment on the left axilla and the control treatment on the right, while the second group receives them the other way round. |
| Product Application. | A two second spray is administered. |
| Sweat Collection | Absorbent cotton pads are used to collect the sweat. On entering the hot room each subject has a pad placed in each of her axillae. After 40 minutes these are removed and rejected. Sweat is then collected for two consecutive periods of 20 minutes, fresh tared pads being used for each collection, and sweat weight determined. |
| Test Design | Subjects attend daily for 3 consecutive days. They receive one treatment with the products each day. On the third day the treatment is immediately followed by a hot room sitting and sweat collection. |
| Analysis of Data | The statistical treatment includes an analysis of variance which allows for subject, side and product effects. The efficacy is calculated from the geometric mean weight of sweat collected from the axillae treated with each product. |

$$\%\ \text{reduction} = 100\,\frac{(C-T)}{C}$$

where C is the geometric mean sweat weight from the axillae treated with the control product and T is the geometric means sweat weight from the axillae treated with the test product. The % reduction is usually calculated for each day separately and for the entire test.

Significance is calculated by applying Student's t-test to the logarithmically transformed weights.

The test product used in Test Method 1 had the composition indicated below.

| Ingredient | % |
|---|---|
| Aluminium chlorhydrate | 4.50 |
| Isopropyl myristate | 6.00 |
| Pyrogenic silica (Aerosil 200) | 0.45 |
| Perfume | 0.44 |
| Propellant[1] | to 100.00 |

[1] $CCl_3F:CCl_2F_2$ — 50:50 by weight.

Test Method II

As Test Method I with the following differences:

Test Product — A solution of treated aluminium chlorhydrate (unless stated otherwise) in water having an aluminium concentration of 2.5% by weight.

Control Product. — A solution of untreated aluminium chlorhydrate (unless stated otherwise) in water having an aluminium concentration of 2.5% by weight.

Method of Application — Approximately 0.5 g of solution was applied to each axilla with a pump-spray applicator.

*Determination of percentage aluminium in polymeric species having a size greater than 100 Ångstroms*

For this purpose there was used a 1.2 m x 6.0 mm column packed with spherical porous silica beads of particle size 75—125 microns, and of surface area 350—500 $m^2/g$, and having a maximum pore size of 100 Ångstroms. The silica employed, available commercially as Porasil AX, had been deactivated to eliminate absorption in molecular size separations. The use of Porasil silica beads as a column packing in chromatography is referred to in "Gel Permeation Chromatography" by K.H. Altgelt and L. Segal, 1971, pages 16—18. The silica was conditioned before use by passage of a single large sample (e.g. 0.2 ml of a 5% w/w solution) of a heat-treated aluminium chlorhydrate. Samples to be tested were made up in deionized water to approximately 0.2 M aluminium and thoroughly dispersed by treatment (4 minutes) with a sonic probe. About 0.2 ml samples of approximately 0.2 M aluminium solutions were applied to the column by a sample loop system and eluted with $10^{-2}$ M aqueous nitric acid solution using a peristaltic pump. A differential refractive index monitor linked to a pen recorder was used to detect fractions as they were eluted. These fractions were collected and analysed for aluminium by atomic adsorption. Complete elution of all aluminium applied in each sample was checked by direct analysis of another sample of the same volume. The percentage of the total aluminium which appeared in the fraction eluted at the void volume of the column was considered as that deriving from polymeric material of a size greater than 100 Ångstroms in effective diameter.

The following Examples illustrate the invention.

Example 1

28 kg of a 50% $^w/_w$ solution of aluminium chlorhydrate which had an Al/Cl molar ratio of 1.99, was added to 42 kg of deionised water in a 75 litre stainless steel reactor internally spray coated with polytetrafluoroethylene and equipped with a propeller stirrer and partial steam jacket. The solution was stirred and heated to 120°C in the closed reactor over a period of 1 hour, then held at this temperature for 4 hours. The treated solution was spray dried in a co-current spray drier using inlet and outlet temperatures of 250°C and 90°C, respectively. The powder was sieved to remove particles greater than 74 microns and tested according to Test Method I (with 26 panellists). The product containing the treated aluminium chlorhydrate gave a 25% reduction in sweat compared with the control product containing a commercially available aluminium chlorhydrate powder ("Microdry Ultrafine"). The result was statistically significant at the 0.1% level. When tested by the chromatographic methods, a solution from the redissolved powder was found to contain 1% of the total aluminium as polymers greater than 100 Ångstroms in effective diameter and 30% of the total aluminium in the Band III fraction.

Example 2

23.9 kg of a 50% $^w/_w$ solution of aluminium chlorhydrate which had an Al/Cl molar ratio of 1.99 was added to 36.0 kg of deionised water at ambient temperature in the reactor described in Example 1. 110.2 g of aluminium chloride hexahydrate was added and the solution stirred to effect complete dissolution. The Al/Cl molar ratio in the final solution was 1.95. The solution was then heated to 120°C in the closed reactor over a period of 1 hour and held at this temperature for 10 hours before cooling to ambient temperature in 1 hour. The treated solution was spray dried in a co-current spray drier using inlet and outlet temperatures of 250°C and 90°C, respectively. The powder was sieved to remove particles greater than 74 microns and tested according to Test Method I (with 40 panellists). The

product containing the treated aluminium chlorhydrate gave an 18% reduction in sweat compared with the control product as in Example 1. The result was statistically significant at the 0.1% level. Upon testing the redissolved powder according to the chromatographic methods, the solution was found to contain 0% of the total aluminium as polymers greater than 100 Ångstroms in effective diameter and 37% of the total aluminium in the Band III fraction.

Example 3

95 g of aluminium chlorhydrate powder having an Al/Cl molar ratio of 2.00 and a water content of 17.1% was mixed with 11.5 g of aluminium chloride hexahydrate and dissolved in deionised water, to give 1 kg of solution. The Al/Cl ratio in the final solution was 1.60. Samples of this solution were placed in 25 ml Pyrex glass screw-cap tubes equipped with polytetrafluoroethylene gaskets and heated to 116°C for 20 hours (the word Pyrex is a trade mark). The solution was cooled to ambient temperature and found to contain 0% of the total aluminium in polymers greater than 100 Ångstroms in effective diameter and 50% of the total aluminium in the Band III fraction. When the treated solution was tested for antiperspirancy against the untreated solution was control according to Test Method II (using a panel of 24 subjects) a reduction of 13% in the sweat collected was observed. The difference was statistically significant at the 5% level. In another test using Test Method II (on a panel of 24 subjects) and a 10% solution of the aluminium chlorhydrate as control, the treated solution gave a reduction of 16% in the sweat collected. The difference was statistically significant at the 2% level.

Example 4

200 g of a 50% $^{w}/_{w}$ solution of aluminium chlorhydrate which had an Al/Cl ratio of 1.99 was added to 800 g of deionised water. Samples of this solution were placed in 25 ml Pyrex glass screw-cap tubes equipped with polytetrafluoroethylene gaskets and maintained at 50°C for 14 days. The solution was cooled to room temperature and found to contain 0% of the total aluminium in polymers greater than 100 Angstroms in effective diameter and 24% of the total aluminium in the Band III fraction.

Example 5

A commercially available basic aluminium chloride solution having a molar ratio of 1.30 was diluted with deionized water to an aluminium concentration of 2.5% by weight. Samples of this solution were placed in 25 ml Pyrex glass screw-cap tubes equipped with polytetrafluoroethylene washers, heated to 120°C for 16 hours, and cooled to ambient temperature. The treated solution was found to contain 0.0% of the total aluminium in polymers greater than 100Å in effective diameter and 65% of the aluminium in the Band III fraction.

The treated solution was tested according to Test Method II (using a panel of 42 subjects) employing a control solution prepared by dilution of the same commercial basic aluminium chloride solution referred to above and which contained 0% of the total aluminium in polymers greater than 100 Ångstroms in effective diameter and 17% of the aluminium in the Band III fraction. Both test products were freshly prepared each day of the antiperspirant test. The treated solution gave a reduction of 19% in the sweat collected compared with the control product. This was statistically significant at the 5% level.

Example 6

Stock solutions were prepared from a 50% by weight aluminium chlorhydrate solution having an aluminium concentration of 12.27% by weight and an Al/Cl molar ratio of 1.97, and aluminium chloride hexahydrate, or a soluble aluminium hydroxide which was 47.0% $Al_2O_3$ by weight, as appropriate, and deionized water. Solutions were prepared at 10.0% aluminium concentration by weight and Al/Cl ratios ranging from 0.9 to 2.2. Ratios lower than 0.9 were prepared at the concentrations required. Heating to a maximum of 40°C with stirring was employed to assist dissolution of the aluminium hydroxide.

Samples were prepared from the stock solutions and deionized water in 25 ml Pyrex glass tubes equipped with screw-caps and polytetrafluoroethylene washers. The tubes were heated in a fan oven for the designated time plus 30 minutes to allow for equilibration of the temperature. At the end of the heating period, the tubes were removed from the oven and cooled rapidly to room temperature in flowing water. The treated samples were analysed immediately for their Band III fraction by the chromatographic procedures described herein. Results of experiments involving reaction times of 24 hours and 6 hours are indicated below in Tables I and II, respectively. In certain places in the tables two values are given, e.g. 77/6. The first value is the percentage of aluminium in the Band III fraction and the second figure is the percentage of aluminium in polymers exceeding 100 Ångstroms. Where only one value is given this is the percentage of aluminium in the Band III fraction, the amount of aluminium in polymers exceeding 100 Ångstroms in these cases being 0%.

TABLE I

| Al/Cl molar ratio \ wt % Al | Temperature 60°C | | | 100°C | | | 120°C | | |
|---|---|---|---|---|---|---|---|---|---|
| | 2.5% | 5.0% | 7.5% | 2.5% | 5.0% | 7.5% | 2.5% | 5.0% | 7.5% |
| 0.7 | 15 | 8 | | 22 | | | | | |
| 0.8 | | | | | | | | 12 | |
| 0.9 | 23 | | | | 12 | | | | |
| 1.2 | 35 | 13 | | 58 | | | 63 | 22 | |
| 1.5 | | | | | 39 | 15 | 75 | | 16 |
| 1.7 | | | | | | | 77/6 | | |
| 1.8 | | 26 | | 79 | | | | 57/1 | 29 |
| 1.9 | | | | | | | | | |
| 2.0 | | | | | 40 | | | | |
| 2.1 | | 15 | 10 | 62/24 | 42/10 | 21 | | | |
| 2.2 | | 12 | | | | | | | 24/43 |

TABLE II

| Al/Cl molar ratio \ wt % Al | Temperature of 120°C 2.5% | 5.0% | 7.5% |
|---|---|---|---|
| 0.7 | 23 | 9 | |
| 0.8 | | | |
| 0.9 | | | 10 |
| 1.2 | | 23 | |
| 1.5 | 78 | | 23 |
| 1.7 | 81 | | |
| 1.8 | 80/1 | | |
| 1.9 | | 53 | |
| 2.0 | | 50/2 | |
| 2.1 | | | 31/8 |
| 2.2 | | | |

The above tables indicate the influence of reaction temperature, Al/Cl molar ratio, and concentration on the production of polymers in the Band III fraction.

For a given Al/Cl molar ratio, reaction temperature and reaction time, the Band III value decreases with increasing concentration. For a given temperature, time and concentration, the Band III value increases to a maximum and then decreases with increasing Al/Cl molar ratio. For a given Al/Cl molar ratio, time and concentration, the Band III value increases with increase in temperature. The tables also show how the temperature and concentration affect the production of polymers exceeding 100 Angstroms in size in relation to the Al/Cl molar ratio.

From numerous experiments that Applicants have conducted including those in the above tables equations have been derived for expressing the percentage aluminium in the Band III fraction resulting from heat treating particular basic aluminium chlorides under certain reaction conditions. Thus for reactions involving compounds having an Al/Cl molar ratio of from 0.6 to 2.2 at temperatures of from 60 to 120°C and aluminium concentrations from 5.0 to 7.5%, and also for the reaction at 60°C and an aluminium concentration of 2.5%, the Band III percentage aluminium value is given by the expression:

$$\%\ \text{Band III Al} = 9.29A - 19.17B + 5.10C + 3.49D - 6.01\ AD + 1.28\ AC - 1.80BC - 2.30\ CD - 11.55\ C^2 - 3.89\ A^2 + 1.89\ B^2 - 1.94\ D^2 + 1.64\ ABD - 5.58\ ACD + 0.56\ BCD - 5.34\ AC^2 + 6.38\ BC^2 - 2.33\ C^2D + 3.94\ C^3 + 2.87\ A^2B - 5.52\ A^2D - 3.19\ A^2C + 0.90\ BD^2 - 2.17\ AD^2 - 1.88\ CD^2 - 0.86\ B^2C + 41.9$$

where A, B, C and D are as follows:

$$A = \frac{T - 100}{25} \qquad B = \frac{[Al] - 5.5}{1.5}$$

$$C = \frac{e^{2R} - 37.5}{26} \qquad D = \frac{t - 19.0}{17.0}$$

where T is the reaction temperature in degrees Centigrade
[Al] is the concentration expressed in percent aluminium by weight
R is the Al/Cl molar ratio
t is the time in hours at temperature T.

For reactions at 100—120°C and at an aluminium concentration of 2.5%, the corresponding expression for compounds having an Al/Cl molar ratio in the range of 0.6 to 2.2, is:

$$\% \text{ Band III Al} = -5.28\,A_1 - 9.58\,B_1 - 1.08\,C_1 - 8.73\,A_1C_1 - 1.98\,A_1B_1 - 5.44\,B_1C_1 - 5.97\,A_1B_1C_1 - 22.92\,B_1^2 - 1.82\,B_1^2C_1 + 7.40\,B_1^3 + 5.84\,A_1C_1^2 + 76.2$$

where $A_1$, $B_1$ and $C_1$ are as follows:

$$A_1 = \frac{T-113}{9.5} \qquad B_1 = \frac{e^R-5.5}{2.5} \qquad C_1 = \frac{t-11.0}{9.0}$$

Comparative Example

An aqueous solution of an aluminium chlorhydrate having an aluminium concentration of 10.0% by weight and an Al/Cl molar ratio of 2.00 was placed in a 25 ml Pyrex glass tube equipped with a screw-cap and a polytetrafluoroethylene washer and heated at 120°C for 24 hours. After cooling to room temperature, the solution was analysed and found to contain 0% of the total aluminium in polymers exceeding 100 Ångstroms in effective diameter and 18% of the aluminium in the Band III fraction.

**Claims**

1. A method of inhibiting perspiration comprising applying to the skin a polymeric antiperspirant active compound which is a basic aluminium chloride, bromide or iodide having an aluminium to chloride, bromide or iodide molar ratio of from 0.5 to 2.5:1 in the form of a powder or an aqueous solution having an aluminium concentration of at least 2.5% by weight, the basic compound being characterised by having at least 20% of the total aluminium in the Band III fraction as measured by the size exclusion chromatography and less than 2% of the total aluminium contained in polymers having a size exceeding 100 Ångstroms.

2. A method as claimed in claim 1, characterised in that the basic aluminium compound has from 25 to 80% of the total aluminium in the Band III fraction.

3. A method of making an antiperspirant product which comprises incorporating an antiperspirant active compound in an applicator for applying the antiperspirant active compound to the skin, characterised in that the antiperspirant active compound is a basic aluminium chloride, bromide or iodide having an aluminium to chloride, bromide or iodide molar ratio of from 0.5 to 2.5:1 in the form of a powder or an aqueous solution having an aluminium concentration of at least 2.5% by weight, the basic compound having at least 20% of the total aluminium in the Band III fraction and less than 2% of the total aluminium contained in polymers having a size exceeding 100 Ångstroms.

4. Method of making an antiperspirant composition, characterised in that the method comprises forming a blend of a basic aluminium chloride, bromide or iodide having an aluminium to chloride, bromide or iodide molar ratio of from 0.5 to 2.5:1, in the form of a powder or an aqueous solution having an aluminium concentration of at least 2.5% by weight with a suitable adjunct or carrier material, the basic aluminium compound having at least 20% of the total aluminium in the Band III fraction and less than 2% of the total aluminium contained in polymers having a size exceeding 100 Ångstroms.

5. Process for improving the antiperspirant activity of a basic aluminium chloride, bromide or iodide having an aluminium to chloride, bromide or iodide molar ratio of from 0.5 to 2.5:1, characterised in that an aqueous solution of the basic aluminium compound having an aluminium concentration of from 2.5 to 8.5% by weight is heated at 50° to 140°C, said molar ratio, concentration and temperature and the time of heating being so chosen that the heat-treated product obtained has at least 20% of the total aluminium in the Band III fraction and less than 2% by weight of the total aluminium in polymers having a size exceeding 100 Ångstroms.

6. An aqueous solution of a basic aluminium chloride, bromide or iodide having an aluminium to chloride, bromide or iodide molar ratio of from 0.5 to 2.5:1 and having an aluminium concentration of 2.5 to 8.5% by weight, the basic aluminium compound having at least 20% of the total aluminium in the Band III fraction and less than 2% of the total aluminium in polymers having a size exceeding 100 Ångstroms.

7. An antiperspirant active agent in powder form consisting of a hydrated basic aluminium chloride, bromide or iodide having an aluminium to chloride, bromide or iodide molar ratio of from 0.5 to 2.5:1 having at least 20% of the total aluminium in the Band III fraction and less than 2% of the total aluminium contained in polymers having a size exceeding 100 Ångstroms.

## Revendications

1. Un procédé pour inhiber la transpiration comprenant l'application à la peau d'un composé polymère antitranspirant actif qui est un chlorure, bromure ou iodure d'aluminium basique à un rapport molaire aluminium/chlorure, bromure ou iodure de 0,5 à 2,5:1 sous forme d'une poudre ou d'une solution aqueuse à une concentration en aluminium d'au moins 2,5% en poids, le composé basique se caractérisant en ce qu'il contient au moins 20% de l'aluminium total dans la fraction de bande III, telle que mesurée par le chromatogramme d'exclusion de dimensions, et moins de 2% de l'aluminium total dans des polymères de dimensions dépassant 100 Å.

2. Un procédé selon la revendication 1, caractérisé en ce que le composé d'aluminium basique contient de 25 à 80% de l'aluminium total dans la fraction de bande III.

3. Un procédé pour préparer un produit antitranspirant qui comprend l'incorporation d'un composé antitranspirant actif dans un applicateur permettant l'application du composé antitranspirant actif sur la peau, caractérisé en ce que le composé antitranspirant actif est un chlorure, bromure ou iodure d'aluminium basique présentant un rapport molaire aluminium/chlorure, bromure ou iodure de 0,5 à 2,5:1, sous forme d'une poudre ou d'une solution aqueuse à une concentration en aluminium d'au moins 2,5% en poids, le composé basique contenant au moins 20% de l'aluminium total dans la fraction de bande III et moins de 2% de l'aluminium total dans des polymères à une dimension dépassant 100 Å.

4. Procédé pour préparer une composition antitranspirante, caractérisé en ce que le procédé comprend la formation d'un mélange d'un chlorure, bromure ou iodure d'aluminium basique présentant un rapport molaire aluminium/chlorure, bromure ou iodure de 0,5 à 2,5:1, sous forme d'un poudre ou d'une solution aqueuse à une concentration en aluminium d'au moins 2,5% en poids avec un produit auxiliaire ou véhicule approprié, le composé d'aluminium basique contenant au moins 20% de l'aluminium total dans la fraction de bande III et moins de 2% de l'aluminium total dans des polymères de dimension dépassant 100 Å.

5. Procédé pour améliorer l'activité antitranspirante d'un chlorure, bromure ou iodure d'aluminium basique présentant un rapport molaire aluminium/chlorure, bromure ou iodure de 0,5 à 2,5:1, caractérisé en ce que l'on chauffe à une température de 50 à 140°C une solution aqueuse du composé d'aluminium basique à une concentration en aluminium de 2,5 à 8.5% en poids, ce rapport molaire, cette concentration, la température et la durée de chauffage étant choisis de manière que le produit obtenu après traitement à la chaleur contienne au moins 20% de l'aluminium total dans la fraction de bande III et moins de 2% en poids de l'aluminium total dans des polymères à une dimension dépassant 100 Å.

6. Une solution aqueuse d'un chlorure, bromure ou iodure d'aluminium basique présentant un rapport molaire aluminium/chlorure, bromure ou iodure de 0,5 à 2,5:1 et à une concentration en aluminium de 2,5 à 8,5% en poids, le composé d'aluminium basique contenant au moins 20% de l'aluminium total dans la fraction de bande III et moins de 2% de l'aluminium total dans des polymères ayant une dimension dépassant 100 Å.

7. Un agent antitranspirant actif sous forme de poudre consistant en un chlorure, bromure ou iodure d'aluminium basique hydraté présentant un rapport molaire aluminium/chlorure, bromure ou iodure de 0,5 à 2,5:1, contenant au moins 20% de l'aluminium total dans la fraction de bande III et moins de 2% de l'aluminium total dans des polymères ayant une dimension dépassant 100 Å.

## Patentansprüche

1. Verfahren zur Schweißverhütung, bei dem auf die Haut eine polymere, schweißverhütend aktive Verbindung aufgebracht wird, die ein basisches Aluminiumchlorid, -bromid oder -jodid mit einem Aluminium/Chlorid-, /Bromid- oder /Jodid-Molverhältnis von 0,5 bis 2,5:1 ist, in Form eines Pulvers oder einer wässrigen Lösung mit einer Aluminiumkonzentration von wenigstens 2,5 Gew.-%, wobei sich die basische Verbindung dadurch auszeichnet, daß sie wenigstens 20% des gesamten Aluminiums in der Bande III—Fraktion, gemessen durch das Größenausschlußchromatogramm, und weniger als 2% des gesamten Aluminiums in Polymeren mit einer Größe über 10 nm (100 Å) hat.

2. Verfahren nach Anspruch 1, dadurch gekennzeichnet, daß die basische Aluminiumverbindung 25 bis 80% des gesamten Aluminiums in der Bande III—Fraktion hat.

3. Verfahren zur Herstellung eines schweißverhütenden Produkts, bei dem eine schweißverhütend wirksame Verbindung in einen Applikator zum Aufbringen der schweißverhütend aktiven Verbindung auf die Haut eingebracht wird, dadurch gekennzeichnet, daß die schweißverhütend aktive Verbindung ein basisches Aluminiumchlorid, -bromid oder -jodid mit einem Aluminium/Chlorid-, /Bromid- oder /Jodid-Molverhältnis von 0,5 bis 2,5:1 ist, in Form eines Pulvers oder einer wässrigen Lösung mit einer Aluminiumkonzentration von wenigstens 2,5 Gew.-%, wobei die basische Verbindung wenigstens 20% des gesamten Aluminiums in der Bande III—Fraktion hat und weniger als 2% des gesamten Aluminiums in Polymeren mit einer Größe über 10 nm (100Å) enthält.

4. Verfahren zur Herstellung eines schweißverhütenden Mittels, dadurch gekennzeichnet, daß das Verfahren die Bildung einer Mischung eines basischen Aluminiumchlorids, -bromids oder -jodids mit

einem Aluminium/Chlorid-, /Bromid- oder /Jodid-Molverhältnis von 0,5 bis 2,5:1 in Form eines Pulvers oder einer wässrigen Lösung mit einer Aluminiumkonzentration von wenigstens 2,5 Gew.-% mit einem geeigneten Zusatz oder Trägermaterial umfaßt, wobei die basische Aluminiumverbindung wenigstens 20% des gesamten Aluminiums in der Bande III—Fraktion hat und weniger als 2% des gesamten Aluminiums in Polymeren mit einer Größe über 10 nm (100 Å) enthält.

5. Verfahren zur Verbesserung der schweißverhütenden Aktivität eines basischen Aluminiumchlorids, -bromids oder -jodids mit einem Aluminium/Chlorid-, /Bromid- oder /Jodid-Molverhältnis von 0,5 bis 2,5:1, dadurch gekennzeichnet, daß eine wässrige Lösung der basischen Aluminiumverbindung mit einer Aluminiumkonzentration von 2,5 bis 8,5 Gew.-% auf 50 bis 140°C erwärmit wird, wobei das Molverhältnis, die Konzentration und die Temperatur und die Zeit des Erwärmens so gewählt werden, daß das erhaltene wärmebehandelte Produkt wenigstens 20% des gesamten Aluminiums in der Bande III—Fraktion hat und weniger als 2 Gew.-% des gesamten Aluminiums in Polymeren mit einer Größe über 10 nm (100 Å) sind.

6. Wässrige Lösung eines basischen Aluminiumchlorids, -bromids oder -jodids mit einem Aluminium/Chlorid-, /Bromid- oder /Jodid-Molverhältnis von 0,5 bis 2,5:1 und mit einer Aluminiumkonzentration von 2,5 bis 8,5 Gew.-%, wobei die basische Aluminiumverbindung wenigstens 20% des gesamten Aluminiums in der Bande III—Fraktion hat und weniger als 2% des gesamten Aluminiums in Polymeren mit einer Größe über 10 nm (100 Å) sind.

7. Schweißverhütend aktives Mittel in Pulverform, bestehend aus einem hydratisierten basischen Aluminiumchlorid, -bromid oder -jodid mit einem Aluminium/Chlorid-, /Bromid- oder /Jodid-Molverhältnis von 0,5 bis 2,5:1 mit wenigstens 20% des gesamten Aluminiums in der Bande III—Fraktion und weniger als 2% des gesamten Aluminiums in Polymeren mit einer Größe über 10 nm (100 Å).